(19) Europäisches Patentamt
European Patent Office
Office européen des brevets

(11) **EP 4 208 121 B1**

(12) **FASCICULE DE BREVET EUROPEEN**

(45) Date de publication et mention
de la délivrance du brevet:
**22.01.2025 Bulletin 2025/04**

(21) Numéro de dépôt: **21772770.0**

(22) Date de dépôt: **02.09.2021**

(51) Classification Internationale des Brevets (IPC):
***A61C 3/02*** *(2006.01)* ***A61C 3/12*** *(2006.01)*
***A61B 17/14*** *(2006.01)*

(52) Classification Coopérative des Brevets (CPC):
**A61C 3/02; A61B 17/144; A61C 3/12;**
A61B 2217/007

(86) Numéro de dépôt international:
**PCT/EP2021/074231**

(87) Numéro de publication internationale:
**WO 2022/049178 (10.03.2022 Gazette 2022/10)**

(54) **SYSTÈME D'ÉQUILIBRAGE POUR MICRO-SCIE**

AUSWUCHTSYSTEM FÜR MIKRO-SÄGE

BALANCING SYSTEM FOR MICRO-SAW

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **04.09.2020 EP 20194548**

(43) Date de publication de la demande:
**12.07.2023 Bulletin 2023/28**

(73) Titulaire: **Bien-Air Holding SA
2504 Bienne (CH)**

(72) Inventeur: **MOSIMANN, Samuel
2520 La Neuveville (CH)**

(74) Mandataire: **BOVARD AG
Patent- und Markenanwälte
Optingenstrasse 16
3013 Bern (CH)**

(56) Documents cités:
**US-A- 3 642 002 US-A1- 2015 066 032**

**Description**

Domaine technique de l'invention

La présente invention se rapporte au domaine des micro-scies destinées à des applications dentaires ou chirurgicales, c'est-à-dire des pièces à main pourvues d'outils de coupe. Elle concerne plus précisément un module d'équilibrage destiné à une telle micro-scie.

État de la technique

On connaît des pièces à main motorisées pourvues d'un moteur intégré générant un mouvement de va-et-vient linéaire d'une scie; le produit « Core Reciprocating Saw » de Stryker est un exemple d'une telle micro-scie pourvue d'un outil de coupe effectuant des mouvements de va-et-vient selon une course d'environ 3 mm et une fréquence comprise entre 10'000 - 30'000 tours par minute selon la vitesse du moteur. Ce produit a du reste fait l'objet d'un brevet américain US4036236 désormais échu.

Selon cette solution, le mouvement rotatif du moteur est transformé en mouvement linéaire réciproque d'un porte-lame via un doigt monté sur des roulements à billes orientés de manière désaxée par rapport à l'axe de transmission.

Un inconvénient de cette solution est la génération d'un niveau élevé de vibrations qui sont transmises directement à la main du praticien en raison du déplacement important et périodique du centre de masse dans la pièce à main. De plus, les vibrations limitent l'efficacité de découpe, qui plafonne avec l'augmentation de la vitesse et de la puissance appliquées au moteur; en effet, à cause de l'augmentation des vibrations, le rendement baisse avec l'augmentation de la puissance. La précision et la vitesse de découpe sont donc limitées par le déplacement du centre de masse du système arbre de transmission-serrage-lame.

Dans le domaine chirurgical, pour la découpe d'os on connaît par ailleurs, par exemple du brevet US5725530, des scies fonctionnant à la manière d'une tronçonneuse, c'est-à-dire basées sur une ou plusieurs bandes flexibles pourvues d'une denture abrasive, et qui sont entraînées en rotation par exemple par paires dans des sens opposés autour de pièces de guidage fixes rigides. Cette solution n'est toutefois pas adaptée pour des opérations de haute précision impliquant des dimensions réduites, et la configuration de l'outil de découpe réduit du reste fortement la maniabilité de l'outil.

Toujours dans le domaine chirurgical, on connaît également des solutions utilisant plusieurs lames entraînées selon des mouvements antagonistes afin de compenser les vibrations, comme par exemple la solution décrite dans la demande internationale WO97/10765. Cette solution est toutefois relativement complexe à mettre en oeuvre et est assez encombrante au niveau de la zone de travail de l'outil.

Le document de brevet US2015/066032 décrit par ailleurs un outil de coupe auquel est imprimé à la fois un mouvement de va-et-vient à basse fréquence et des vibrations ultrasoniques à très haute fréquence. Un élément de conversion pourvu de sillons obliques dans lesquels sont positionnées des goupilles d'entraînement permettent d'imprimer un mouvement de va-et-vient respectivement à un cylindre d'entraînement d'une lame et conjointement à un cylindre de contre-masse. Un inconvénient de ce type d'outil est toutefois que le dispositif d'équilibrage intégré ne permet aucun découplage entre la partie moteur et celle d'entraînement de la lame.

La solution divulguée dans le document US3642002, beaucoup plus ancien, concerne quant à elle un outil chirurgical pourvu d'un système de bielle reliée au rotor solidaire d'un arbre moteur, et dans laquelle le rotor est pourvu d'un contrepoids. Un inconvénient d'une telle solution est d'intégrer la contre-masse directement dans la partie moteur, ce qui en affecte les performances et empêche ici encore toute configuration modulaire du dispositif d'équilibrage. Il existe par conséquent un besoin pour une solution exempte de ces limitations connues.

Résumé de l'invention

Un but de la présente invention est de proposer une nouvelle micro-scie à usage dentaire ou chirurgical dont l'équilibrage soit réalisé de façon simple et efficace, et permettant par ailleurs un découplage de la partie moteur par rapport à la pièce à main.

Un autre but de la présente invention est de proposer une nouvelle micro-scie à usage dentaire ou chirurgical d'encombrement minimal, et nécessitant le minimum d'adaptations par rapport aux solutions existantes.

Selon l'invention, ces buts sont atteints grâce à un dispositif de découpage à usage chirurgical ou dentaire comprenant un moteur et une pièce à main équipée d'un module de transmission pour un outil de coupe, le moteur étant relié à la pièce à main, et le module de transmission comprenant:

- une partie d'accouplement moteur agencée pour être entraînée en rotation par le moteur;

- une chaîne cinématique transformant le mouvement rotatif du moteur en un premier mouvement de va-et-vient linéaire d'un porte-lame via un arbre de transmission rotatif pourvu d'un excentrique.

Le module de transmission est caractérisé en ce qu'il comprend par ailleurs une contre-masse agencée de manière à se déplacer selon un deuxième mouvement de va-et-vient linéaire synchronisé sur le premier mouvement de va et vient linéaire du porte-lame, et dans le sens opposé à ce dernier.

Un avantage de la solution proposée est de réduire le niveau de vibrations sans parallèlement nécessiter de mise en oeuvre d'une solution complexe, ni de générer d'encombrement additionnel significatif tant au niveau de la pièce à main que de la zone de travail de l'outil de coupe.

Un autre avantage de la solution proposée est d'offrir une construction modulaire d'un système de transmission-conversion garantissant à la fois une force de connexion suffisante entre la pièce-à-main et le moteur au niveau opérationnel, mais également un démontage aisé pour utiliser la partie moteur en combinaison avec d'autres outils chirurgicaux.

Selon un mode de réalisation préférentiel, la première chaîne cinématique du module de transmission comprend un premier doigt d'entraînement pour le porte-lame monté sur une première partie de l'excentrique, ainsi qu'un deuxième doigt d'entraînement de la contre-masse monté sur une deuxième partie de l'excentrique.

Un avantage de cette solution est qu'elle peut être intégrée aisément à des solutions existantes, via simplement un doublement des doigts, et sans devoir créer de mécanismes de transmission parallèle pour la contre-masse, ce qui pourrait être à l'origine de vibrations parasites ne réduisant pas efficacement les vibrations, mais étant susceptibles au contraire de les augmenter.

Selon un mode de réalisation encore plus préférentiel, l'excentrique est agencé symétriquement en forme de « V », le premier doigt d'entraînement du porte-lame est monté sur une première série de roulements autour de la première partie de l'excentrique et le deuxième doigt d'entraînement du porte-lame est monté sur une deuxième série de roulements autour de la deuxième partie de l'excentrique.

Un avantage de cette solution est de pouvoir utiliser une forme usuelle d'excentrique dans la chaîne cinématique de transmission et de ne devoir simplement rajouter qu'un deuxième doigt monté symétriquement sur une série de roulements pour générer le mouvement synchronisé inverse de la contre-masse. Ainsi l'intégration du système d'actionnement de la contre-masse est rendue particulièrement simplifiée sur la même chaîne cinématique et avec un encombrement minimal.

Selon un autre mode de réalisation préférentiel, la forme géométrique de la contre-masse est adaptée à celle de l'outil de coupe et du porte-lame, ce qui confère une flexibilité maximale dans le design du système d'équilibrage. De préférence, la contre-masse sera ainsi cylindrique pour correspondre à un porte-lame sous forme d'arbre lui aussi cylindrique.

Selon un autre mode de réalisation préférentiel, la contre-masse est guidée axialement selon un axe de transmission correspondant vectoriellement au sens de déplacement longitudinal du porte-lame et de l'outil de coupe par au moins un élément de guidage axial inséré dans au moins un orifice de guidage pratiqué dans la contre-masse.

Un avantage technique de cette solution est de pouvoir compenser au mieux les vibrations générées par les mouvements de va-et-vient de l'outil de coupe entraînée par le porte-lame, lequel est en général lui aussi guidé selon cette direction. Les vibrations sont ainsi confinées dans une seule et même direction, et l'opération d'auto-équilibrage est plus facile sans devoir maîtriser des mouvements parasites.

Selon une variante encore plus préférentielle correspondant à ce mode de réalisation, l'élément de guidage axial est formé par un premier rail de guidage axial et un deuxième rail de guidage axial reliant parallèlement une première pièce du châssis connectée au moteur à une deuxième pièce du châssis supportant et guidant axialement le porte-lame du module de transmission, le premier rail de guidage axial et le deuxième rail de guidage axial étant insérés dans des orifices de guidage respectifs de la contre-masse.

L'avantage technique additionnel de cette variante est de pouvoir maximiser les propriétés de guidage de la contre-masse, d'une part, et d'autre part de libérer de la place au niveau central pour y loger un sabot d'accouplement pour le porte-lame. Par ailleurs, le fait que les rails fassent à la fois office de goupilles de guidage pour la contre-masse et d'élément de liaison du châssis du module de transmission permet de réduire le nombre de pièces nécessaires à sa réalisation, et donc de réduire les coûts de fabrication.

Selon encore un autre mode de réalisation préférentiel, le dispositif de découpage à usage chirurgical ou dentaire selon l'invention est caractérisé en ce que le module de transmission comprend par ailleurs un dispositif de refroidissement pour la contre-masse.

Un avantage de cette solution est de pouvoir optimiser l'équilibrage du dispositif obtenu via la contre-masse en minimisant l'effet indésirable du réchauffement résultant des frottements lorsque l'énergie cinétique du système global augmente.

Selon un mode de réalisation encore plus préférentiel pour réaliser un tel dispositif de refroidissement, le premier rail de guidage axial comprend un premier conduit de refroidissement intégré et/ou le deuxième rail de guidage axial comprend un deuxième conduit de refroidissement intégré.

Un avantage de cette solution est de pouvoir combiner directement le mécanisme de refroidissement dans les éléments de guidage, ce qui minimise l'encombrement global du système qui ne nécessite ainsi aucune pièce dédiée pour réaliser cette fonction technique supplémentaire de refroidissement, qui est réalisée par des éléments directement intégrés au châssis et qui permettent ainsi de remplir simultanément d'autres fonctions.

Selon encore un autre mode de réalisation préférentiel, le module de transmission est connecté de façon amovible au moteur, et le poids de la contre-masse est déterminé de manière à être supérieur à une valeur minimale définie par rapport au poids combiné du porte-lame et de l'outil de coupe en fonction de la force de déconnexion du module de transmission au moteur, de la course de l'outil de coupe, et de la fréquence d'oscillation du mouvement de va-et-vient de l'outil de coupe.

Selon un autre mode de réalisation préférentiel, le poids de la contre-masse est déterminé de manière à être inférieur à une valeur prédéfinie déterminée par un bilan thermique.

Un avantage de chacune de ces deux solutions est qu'elles permettent de définir des contre-masses dont le poids ne soit pas nécessairement égal, mais de préférence inférieur à l'ensemble formé par l'outil de coupe et le porte-lame. Il est ainsi possible de réduire les dimensions de cette contre-masse et minimiser l'encombrement global d'un tel système auto-équilibré, tout en maximisant le niveau de conception du système selon les besoins, selon que l'on privilégie par exemple la fiabilité du couplage moteur par rapport au niveau d'échauffement à l'usage en fonction des opérations à réaliser.

Ainsi selon encore un autre mode de réalisation particulièrement préférentiel, le poids de la contre-masse est simplement compris entre 25% et 50% du poids combiné de l'ensemble formé par le porte-lame et l'outil de coupe, ce qui constitue un optimum en termes de fiabilité d'accouplement et d'échauffement à l'usage.

Selon encore un autre mode de réalisation préférentiel, la densité de la contre-masse est supérieure à 7000 kg par mètre cube, ce qui permet de réduire encore davantage le volume de celle-ci et donc son encombrement pour un poids donné, et par suite de minimiser le niveau de frottements et d'échauffement généré à l'usage.

Selon encore un autre mode de réalisation préférentiel, la partie d'accouplement moteur du module de transmission consiste en un orifice cannelé, lequel coopère avec un nez d'accouplement standard dudit moteur comportant une série de rainures dans lesquelles sont insérées des O-rings.

Une telle configuration du module de transmission permet un accouplement avec n'importe quel micromoteur dentaire ou chirurgical standard, sans nécessiter de quelconques adaptations de ces derniers. Ainsi la solution proposée résout des problèmes d'incompatibilités éventuelles qui auraient pu survenir.

Par ailleurs, puisque précisément le module de transmission est compatible avec tout type de micromoteur usuel, et qu'une pièce à main comprenant un tel module de transmission peut donc être vendues indépendamment des parties « moteurs » auxquelles elles peuvent être aisément couplées et découplées, la présente invention concerne aussi de telles pièces à main pourvues de tels modules de transmission prises indépendamment du moteur.

Brève description des dessins

D'autres caractéristiques avantageuses ressortiront plus clairement de la description qui suit d'un mode particulier de réalisation de l'invention donné à titre d'exemple non limitatif et représenté par les dessins annexés, dans lesquels:

- la figure 1 illustre une vue de profil des parties fonctionnelles d'un outil de découpage chirurgical ou dentaire selon la présente invention, à savoir le micromoteur et la pièce à main / micro-scie;

- les figures 2A et 2B illustrent respectivement une vue de profil en trois dimensions d'un micromoteur usuel destiné à des applications dentaires ou chirurgicales ainsi qu'une vue en coupe sagittale de son nez d'accouplement;

- la figure 3 est une vue en perspective d'un outil de découpage à usage chirurgical ou dentaire selon un mode de réalisation préférentiel pour l'invention, pourvu d'un module de transmission équilibré par une contre-masse courte;

- la figure 4 est une vue en perspective d'un module de transmission pour outil de découpage à usage chirurgical ou dentaire selon un autre mode de réalisation préférentiel pour l'invention, dans lequel la contre-masse est longue;

- la figure 5 est une vue en perspective éclatée du module de transmission pour outil de découpage à usage chirurgical ou dentaire selon le mode de réalisation préférentiel de la figure 3;

- la figure 6 est une vue en coupe sagittale du module de transmission illustré sur les figures 3 et 4;

- les figures 7A et 7B sont respectivement une vue de dessus et en coupe sagittale de l'arbre de transmission, l'excentrique et les doigts pour l'activation respectivement du porte-lame et de la contre-masse selon le mode de réalisation préférentiel décrit à l'aide des figures précédentes ;

- la figure 8 est une vue de profil en trois dimensions d'un module de transmission pour outil de découpage à usage chirurgical ou dentaire selon un mode de réalisation préférentiel avec contre-masse longue, et qui est pourvu par ailleurs d'un mécanisme de refroidissement intégré.

<u>Description détaillée</u>

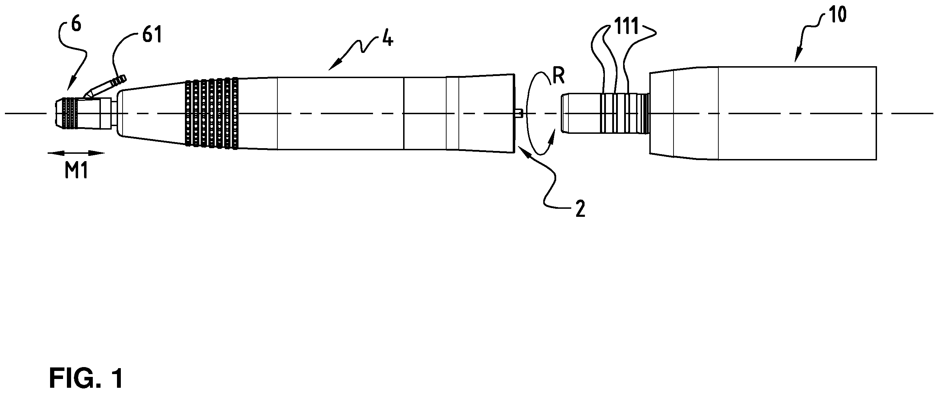

La figure 1 montre une vue de côté des parties fonctionnelles d'un outil de découpage chirurgical ou dentaire selon la présente invention, et comprend, comme pour une pièce à main dentaire classique, une première partie renfermant le moteur et une autre constituant la pièce à main proprement dite, c'est-à-dire la partie qui est manipulée par le chirurgien-dentiste.

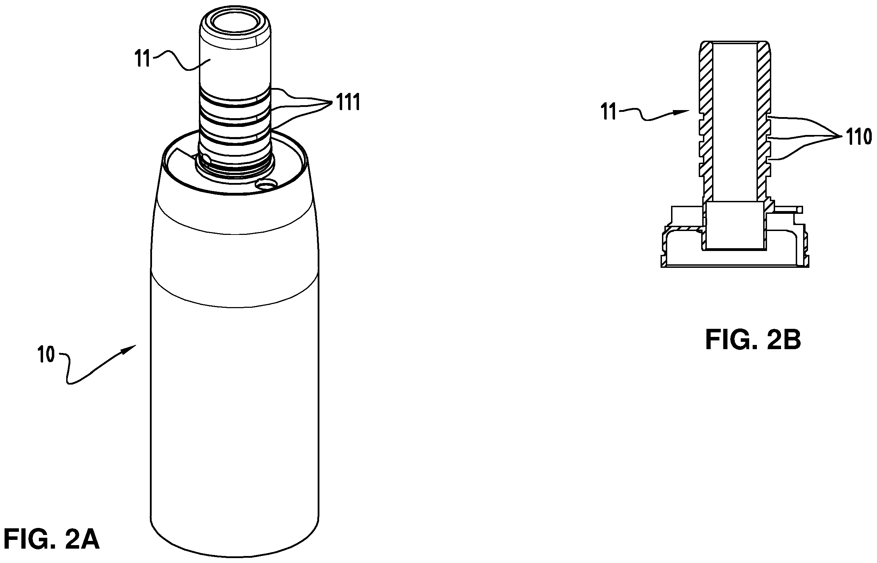

Ainsi sur la figure 1 on peut distinguer, sur la droite de la figure, le micromoteur 10, ainsi que son nez d'accouplement 11 comprenant classiquement une série de rainures dans lesquelles sont disposées des O-rings pour la transmission d'un mouvement rotatif matérialisé par la flèche R sur cette même figure. La figure 2A représente un tel micromoteur 10 en perspective, et qui ressemble en tous points à un micromoteur 10 usuel pour pièce à main avec un nez d'accouplement court 11 sur lequel sont disposées des O-rings 111 logées dans la série de stries 110 visible sur la figure 2B qui montre le détail du nez d'accouplement en coupe sagittale.

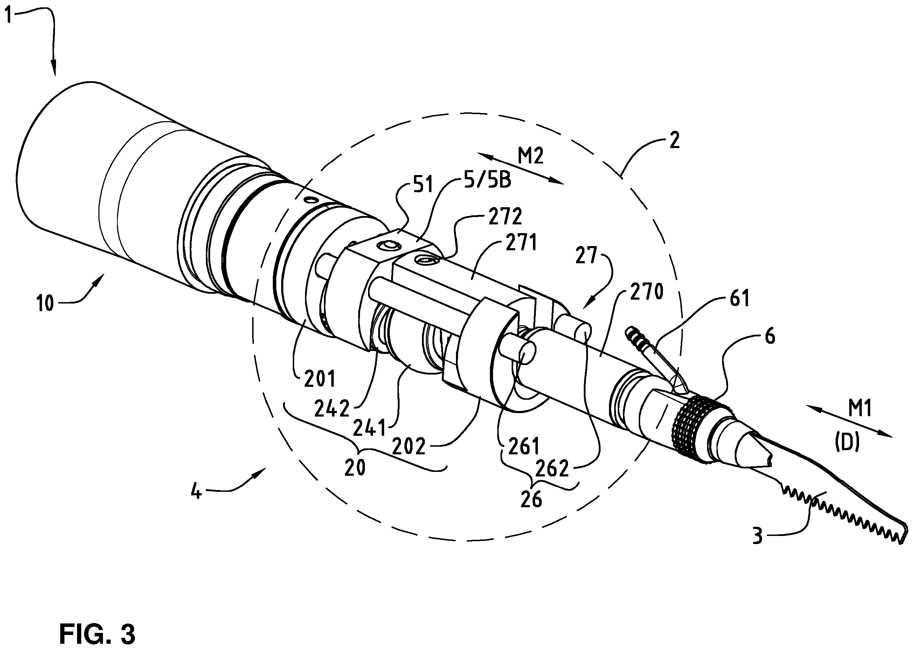

Sur la gauche de la figure 1, on peut distinguer le carter de la pièce à main 4, c'est-à-dire son enveloppe extérieure, renfermant le module de transmission 2 décrit plus loin. Ce module de transmission 2, permet de transformer le mouvement rotatif imposé à l'entrée de la pièce à main 4 en un mouvement de va-et-vient M1 d'un outil de coupe, non représenté sur cette figure, et que l'on qualifiera dans ce qui suit de « premier » mouvement de va-et-vient M1 comme correspondant à celui du porte-lame 27, c'est-à-dire l'élément portant l'outil de coupe 3. Ces deux éléments sont représentés plus loin sur la figure 3 montrant une vue d'ensemble du mécanisme de transmission et d'auto-équilibrage selon un mode de réalisation préféré pour la présente invention.

A l'extrémité gauche de la pièce à main 4 est représenté un dispositif de serrage 6 pour l'outil de coupe 3, qui est pourvu d'un aileron correspondant à un canal d'irrigation intégré 61 destiné à approvisionner la zone traitée chirurgicalement 61 en liquide physiologique, de manière à minimiser l'échauffement sur cette zone. La solution de couplage d'un outil de coupe à un mécanisme de transmission telle que décrite par exemple dans le brevet européen EP2316356 de la demanderesse peut par exemple très bien convenir pour le montage de l'outil de coupe 3 dans le cadre de la présente invention, et pour cette raison, aucun détail supplémentaire ne sera fourni dans ce qui suit concernant ces aspects de fixation de l'outil de coupe 3 au module de transmission 2 de la pièce à main 4.

La construction modulaire ainsi proposée, avec une pièce à main 4 portant l'outil de coupe 3 pour former une micro-scie amovible par rapport au micromoteur 10, offre donc d'une part la possibilité d'utiliser le même micromoteur 10 chirurgical non seulement en combinaison avec la pièce-à-main 4, mais également avec toute autre pièce-à-main mécanique disponible dans un hôpital ou cabinet médical (par exemple des contre-angles d'implantologie); d'autre part, elle permet d'assurer un processus de nettoyage, de décontamination et de stérilisation plus efficace de tous les éléments de la pièce-à-main 4, notamment des composants en contact avec le liquide d'irrigation, donc en contact indirect avec le patient. En fait, la possibilité de déconnecter la pièce-à-main 4 du moteur permet de mieux nettoyer manuellement la pièce-à-main 4 ou d'utiliser des posages dédiés lors des nettoyages dans une machine à laver ou dans un thermo-désinfecteur.

La figure 3 illustre plus en détail le mécanisme de transmission selon un mode de réalisation préférentiel pour l'invention, dans lequel la contre-masse 5 utilisée est une contre-masse courte 5B, dont l'objectif est ainsi de minimiser les frottements et la génération d'un échauffement trop important pour réaliser l'auto-équilibrage. Elle consiste en une vue en perspective en trois dimensions d'un ensemble complet composé sur le micromoteur 10, assemblé à une pièce à main 4 dont le carter a été supprimé pour voir l'intérieur et en particulier le module de transmission 2, encerclé en pointillés, qui est destiné à transmettre le mouvement du moteur à l'outil de coupe 3 selon un premier mouvement de va-et-vient M1 selon une course D prédéterminée.

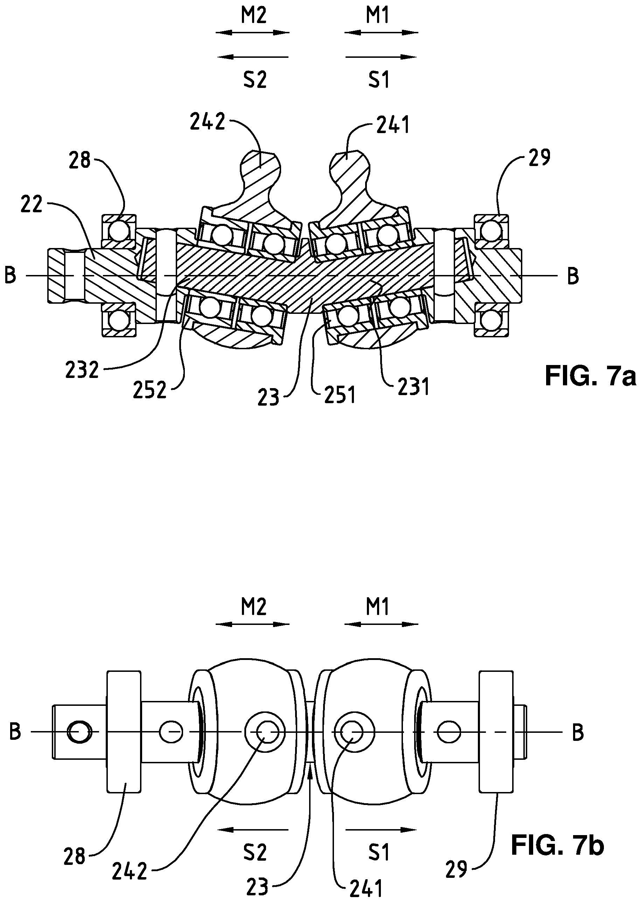

L'outil de coupe 3 est entraîné selon un mouvement de va-et-vient en translation par un porte-lame 27, et est fixé à ce dernier par l'intermédiaire d'un dispositif de serrage 6 pourvu d'un canal d'irrigation 61 intégré approvisionnant la zone de traitement chirurgicale en liquide physiologique. La transformation du mouvement de rotation du moteur en un mouvement de translation du porte-lame 27 est réalisée via un premier doigt 241, dont la structure sera détaillée notamment à l'aide des figures 7a et 7b, et qui est insérée dans un premier orifice d'insertion 272 d'un sabot d'accouplement 271 prévu à cet effet. La pièce de liaison du porte lame 27 entre le sabot d'accouplement 271 et le dispositif de serrage est quant à elle constituée par un corps cylindrique 270.

Comme on peut le constater sur la figure 3, le module de transmission comprend un châssis 20 formé par une première pièce 201 de liaison avec le micromoteur 10, et une deuxième pièce 202 en forme de U et constituant le support du porte lame 27. La première pièce 201 et la deuxième pièce 202 sont reliées entre elles à l'aide respectivement de deux tiges cylindriques, formant parallèlement un premier rail de guidage axial 261 et d'un deuxième rail de guidage axial 262, constituant un élément de guidage axial 26 pour la contre-masse 5 (ici la contre-masse courte 5B). De cette manière, le châssis 20 peut réaliser une fonction de guidage pour la contre-masse 5 sans nécessiter l'ajout d'une pièce dédiée. La contre-masse courte 5B est entraînée selon un deuxième mouvement de va-et-vient M2, opposé à celui du premier mouvement de va-et-vient M1 de l'outil de coupe, via un deuxième doigt 242 qui est inséré dans un deuxième orifice 51 de la contre-masse 5.

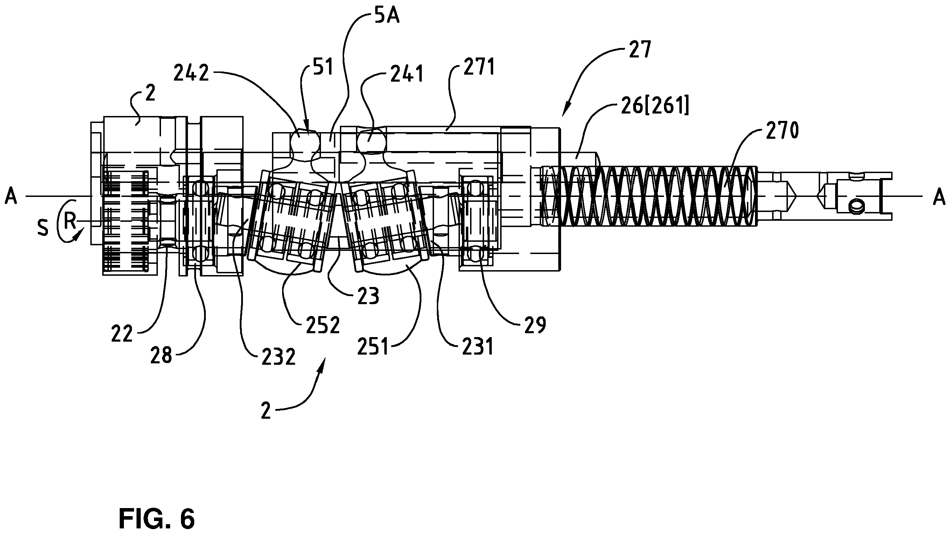

Ainsi, par rapport à une solution de l'art antérieur, l'auto-équilibrage a été réalisé simplement grâce à un doublement des doigts, sans devoir toutefois modifier substantiellement ni la structure du châssis, du mécanisme d'entraînement, et notamment de l'excentrique 23 illustré notamment sur les figures 6 et 7a/7b. Cet excentrique, rotatif autour du même axe que celui de l'arbre de transmission 22, génère un mouvement de va-et-vient en translation de l'extrémité des doigts tout en provoquant un mouvement de rapprochement et respectivement d'éloignement de leur extrémité inférieure en raison de l'écartement non constant vis-à-vis de l'axe de rotation tout le long de l'excentrique.

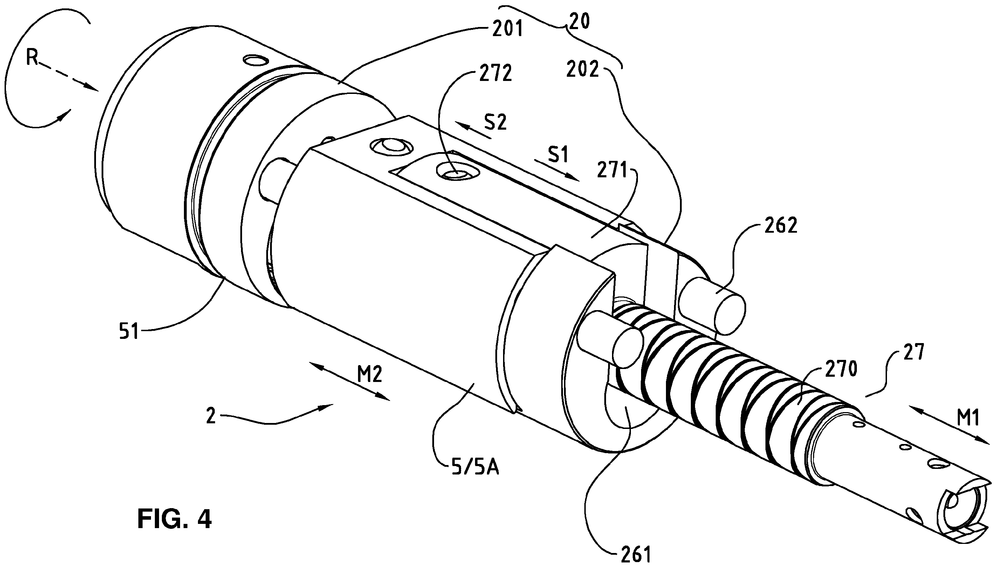

La figure 4 illustre un agrandissement d'un module de transmission 2 selon un autre mode de réalisation préférentiel pour l'invention, utilisant cette fois une contre-masse longue 5A au lieu de la contre-masse courte 5B illustrée sur la figure 3 précédente. Les autres pièces illustrées sur cette figure sont en tous points identiques à celles de la figure 3 et ne seront pas redécrites en détail. Le mécanisme de transmission et d'équilibrage reste identique à celui du mode de réalisation précédent en permettant de générer le premier mouvement de va-et-vient M1 du porte-lame 27 à partir du mouvement rotatif R fourni en entrée via le micromoteur 10, et de générer parallèlement un deuxième mouvement de va-et-vient M2 de la contre-masse longue 5A. Le deuxième mouvement de va-et-vient M2 de la contre-masse est synchronisé en opposition de phase par rapport au premier mouvement de va-et-vient M1; ces deux mouvements M1 et M2 étant toujours générés via respectivement un premier doigt 241 et un deuxième doigt 242, dont seules les extrémités sont visibles, le reste étant caché sous la contre-masse longue 5A. Le sens de déplacement du premier doigt S1 est toujours opposé à celui de déplacement du deuxième doigt S2.

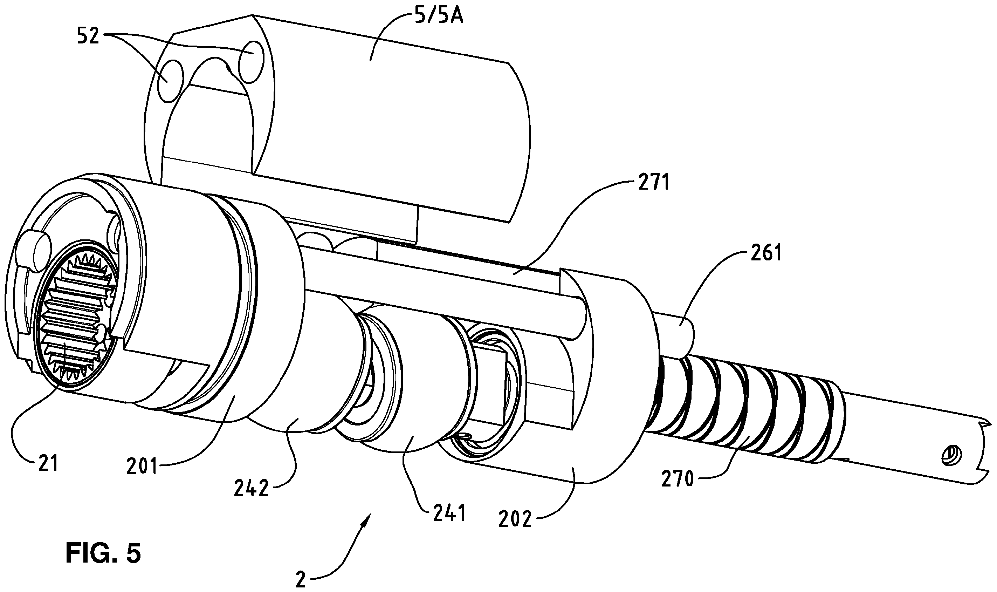

La figure 5 constitue une vue en perspective éclatée du module de transmission 2 pour outil de découpage à usage chirurgical ou dentaire selon le mode de réalisation préférentiel de la figure 4 précédente, c'est-à-dire possédant une contre-masse longue 5B. On y voit désormais le corps de chacun des doigts d'entraînement, c'est-à-dire le premier doigt 241 d'entraînement du porte-lame 27, et le deuxième doigt 242 d'entraînement de la contre-masse 5A, qui possède par ailleurs deux orifices de guidage 52 longitudinaux permettant l'insertion respective de chacun des rails de guidage axiaux orientés parallèlement à l'axe de déplacement du porte-lame 27, dont on peut voir le corps cylindrique 270 et le sabot de couplage 271. Sur cette figure, seul le premier rail de guidage axial 261 est visible. Cette figure met par ailleurs en évidence l'orifice de couplage 21 cannelé du module de transmission 2 qui est destiné à coopérer avec le nez d'accouplement 11 du moteur pour imprimer le mouvement de rotation à un arbre de transmission, illustré sur les figures suivantes, et constitue ainsi une variante structurelle préférée pour la réalisation de la partie d'accouplement moteur du module de transmission 2.

La figure 6, qui constitue une vue en coupe sagittale du module de transmission qui peut être utilisé dans le cadre des modes de réalisation préférentiels des modules de transmission illustrés sur les figures 3 et 4, matérialise un premier axe longitudinal A-A de déplacement de l'outil de coupe 3 via le porte-lame 27. Le mouvement rotatif R du micromoteur 10 est transmis à un arbre de transmission 22 rotatif, qui s'étend selon un deuxième axe longitudinal B-B parallèle au premier axe longitudinal A-A. Ce deuxième axe longitudinal n'est pas illustré sur cette figure pour des questions de lisibilité, mais visible notamment sur les figures 7a et 7b décrites dans ce qui suit. L'arbre 22 de transmission est monté rotatif entre des roulements d'entrée 28 et des roulements de sortie 29, solidaires respectivement de la première pièce 201 et de la deuxième pièce 202 du châssis 20 du module de transmission 2. Par ailleurs, un excentrique 23 configuré symétriquement en forme de « V » est monté sur cet arbre de transmission 22 rotatif. L'excentrique 23 comporte une première partie 231 sur laquelle est montée le premier doigt 241, dont le mouvement de va-et-vient entraîne le porte-lame 27, et par suite, l'outil de coupe 3, et une deuxième partie 232 sur laquelle on a monté le deuxième doigt 242 prévu pour l'entraînement de la contre-masse 5 en vue de réaliser l'auto-équilibrage du système. Sur cette figure, on peut bien distinguer le deuxième orifice d'insertion 51 agencé dans la contre-masse 5 pour le deuxième doigt 242. Afin de permettre de pouvoir transformer le mouvement de rotation de l'excentrique 23 en un mouvement de va-et-vient des doigts, le corps de chacun de ces doigts est par ailleurs pourvu d'une série de roulements, à savoir une première série de roulements 251 pour le premier doigt 241, et de façon correspondante, une deuxième série de roulements 252 pour le deuxième doigt.

Le mécanisme de transmission, ou plus exactement de conversion du mouvement rotatif appliquée en entrée en mouvement de va-et-vient en translation est expliqué en détail à l'aide des figures 7a et 7b, qui montre respectivement une vue en coupe sagittale et de dessus de l'arbre de transmission 22 pourvu de l'excentrique et des doigts montés dessus.

Comme on peut le voir sur la figure 7a, l'arbre de transmission 22 tourne autour de son axe, c'est-à-dire le deuxième axe longitudinal B-B. Il est monté rotatif par rapport au châssis 20 via respectivement les roulements d'entrée 28 vis-à-vis de la première pièce du châssis 201 et les roulements de sortie 29 vis-à-vis de la deuxième pièce 202 du châssis 20 du module de transmission 2. Lors de sa rotation, il entraîne l'excentrique 23, dont la première partie 231 sur laquelle est monté le premier doigt 241 via la première série de roulements 251 va générer un premier mouvement de va-et-vient M1 dans un premier sens S1, alors que la deuxième partie sur laquelle est monté le deuxième doigt 242 via la deuxième série de roulements 252 va générer un deuxième mouvement de va-et-vient M2 d'amplitude identique, mais de sens simplement opposé (c'est-à-dire selon le deuxième sens S2). La figure 7b met en évidence les doigts vus de dessus ; en dehors des roulements qui ne sont plus visibles à l'intérieur de ceux-ci tous les éléments illustrés sont identiques à ceux de la figure 7a. Cette vue permet de se représenter aisément le mouvement en translation de chacun de ces doigts le long du deuxième axe longitudinal B-B et permet d'expliquer pourquoi il est plus intéressant de coupler l'outil de coupe 3, via le porte-lame 27, par l'intermédiaire du premier doigt 241 et de réaliser l'équilibrage à l'aide de la contre-masse 5 via le deuxième doigt 242. En effet, étant donné que l'outil de coupe 3 est agencée à l'extrémité de l'outil de découpage, il est plus naturel de disposer le sabot de couplage au niveau de cette même extrémité, soit le plus à droite possible sur ces figures.

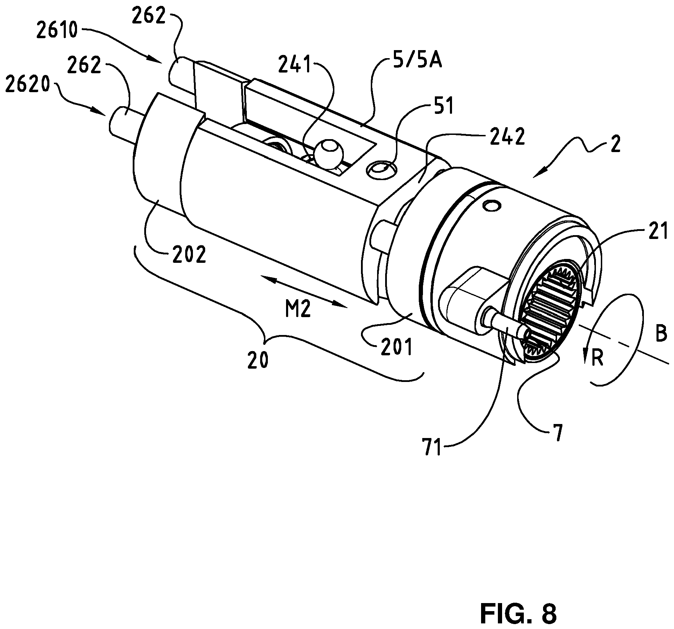

La figure 8 illustre une variante particulièrement préférée d'un module de transmission 2 pour la réalisation de la présente invention, dont le châssis 20 est pourvu d'un mécanisme de refroidissement 7 intégré pour la contre-masse 5. Selon ce mode de réalisation illustré utilisant une contre-masse longue 5B, qui vise à réaliser le plus efficacement possible l'équilibrage du dispositif de découpage proposé en fonctionnement, et garantir au mieux l'accouplement du micromoteur - non représenté sur cette figure mais dont le mouvement de rotation R représenté ici sur la droite de la figure 8 - vis-à-vis de la pièce à main 4 via l'orifice de couplage 21. Selon la variante préférentielle illustrée, le dispositif de refroidissement intégré 7 utilise avantageusement les éléments de liaison longitudinaux entre la première pièce du châssis 201 et la deuxième pièce du châssis 202, c'est-à-dire les poutres cylindriques qui forment parallèlement des éléments de guidage axial 26, en y incorporant des conduits de refroidissement intégrés. Ainsi, comme on peut les voir représentés en pointillés sur la figure 8, le premier rail de guidage axial 261 est pourvu d'un premier conduit de refroidissement intégré 2610 et similairement le deuxième rail de guidage axial 262 est pourvu d'un deuxième conduit de refroidissement intégré 2620. Un conduit d'alimentation de fluide 71 peut être ainsi directement aménagé à l'extrémité du châssis proche du micromoteur, et être utilisé directement pour un approvisionnement en liquide physiologique, en remplacement par exemple du canal d'irrigation 61 visible notamment sur la figure 3. Bien que selon le mode de réalisation préféré sur cette figure on utilise deux canaux pour les conduits de refroidissement, on comprendra qu'une telle configuration est uniquement suffisante, mais pas nécessaire pour la mise en oeuvre de la présente invention, et qu'il serait également envisageable de n'utiliser qu'un seul conduit de refroidissement intégré dans un élément de guidage axial 26 qui ne comprendrait qu'un seul rail de guidage.

La réalisation d'une ou plusieurs canalisations d'approvisionnement à l'intérieur des rails de guidage de la contre-masse 5, qui deviennent donc des tubes d'irrigation, présente deux avantages particulièrement intéressants au niveau opérationnel:

- le liquide d'irrigation permet de refroidir localement la zone de frottement de la contre-masse 5, permettant ainsi d'utiliser des contre-masses plus lourdes - comme par exemple la contre-masse longue illustrée sur la figure 8 - et donc des lames chirurgicales plus longues et lourdes ;
- l'irrigation de l'outil de coupe via un mécanisme interne permet d'éviter de devoir connecter une ligne d'irrigation au porte-lame, comme c'est le cas habituellement une connexion de la ligne d'irrigation qui est donc réalisée à la fois plus proche des doigts du chirurgien, et plus proche de la zone de travail, ce qui implique aussi un risque de déconnexion accidentelle de la ligne.

Ainsi, cette solution confère une amélioration significative de l'ergonomie du chirurgien et de la sécurité du patient, tout en procurant une flexibilité accrue quant au calibrage de l'outil en fonction des besoins.

Toujours selon un mode de réalisation préférentiel pour la réalisation de la présente invention, le poids de la contre-masse 5 est compris entre une valeur minimale, dépendante de la force de déconnexion moteur-pièce-à-main, de la fréquence de va-et-vient maximale de l'outil de coupe 3, de la course D de l'outil de coupe illustrée par exemple sur la figure 3, et de la masse de l'ensemble outil de coupe 3 - porte-lame 27 et une valeur maximale, dépendant de la puissance de refroidissement de la micro-scie / pièce à main 4 et du diamètre de préhension de l'outil de coupe. Ainsi, le poids de la contre-masse n'est pas nécessairement égal au poids de l'ensemble solidaire de l'outil de coupe et qui est entraînée selon un mouvement de va-et-vient.

Ces contraintes sont formulées dans l'équation 1 qui suit, qui reprend l'équation fondamentale de la dynamique suivant une modélisation conformément à un système matérialisé selon des lois de physique relevant de la mécanique du point. Plus particulièrement, la masse $M_c$ de la contre-masse 5 doit être supérieure à une valeur limite définie par l'équation (1) suivante:

$$x = \frac{\delta}{2}\cos(2\pi f t) \rightarrow \frac{d^2 x}{dt^2} = \frac{\delta}{2}(2\pi f)^2 \cos(2\pi f t)$$

$$a_{max} = 2\delta\pi^2 f^2$$

$$F_d > [M_s + M_p]\, a_{max} - M_c\, a_{max}$$

$$M_c > M_s + M_p - F_d / 2\delta\pi^2 f^2 \quad (1)$$

où $M_s$ est la masse de l'outil de coupe 3, $M_p$ celle du porte-lame 27, c'est-à-dire du corps cylindrique 270 et du sabot d'accouplement 271, $F_d$ la force de déconnexion, $\delta$ la course D de l'outil de coupe 3, et f la fréquence maximale du mouvement de va-et-vient.

L'équation (1) ci-dessus permet d'assurer que l'outil de coupe 3 ne soit pas déconnectée du module moteur, c'est-à-dire la pièce correspondant au micromoteur 10, accidentellement lors des différentes situations de travail. Pour ceci, on calcule l'accélération maximale du système et déduit de l'équation indiquant que la somme des forces appliquées au système est égale à la masse multipliée par l'accélération.

La modélisation du système porte-lame 27 et outil de coupe 3 d'une part, et contre-masse 5 d'autre part comme des points matériels est justifiée par l'hypothèse que les composants solides sont soumis à des vibrations de très faible amplitude, et à haute fréquence, et par conséquent leur comportement dynamique de basse fréquence est essentiellement donné par le mouvement de leur centre de gravité. Puisque le seul degré de liberté actif consiste en le mouvement longitudinal le long de l'élément de guidage axial 26 (formé de préférence par les deux rails de guidage axiaux 261,262), il n'y a pas d'effet d'entraînement en rotation qui demanderait de prendre en compte la position du centre de gravité par rapport à un éventuel centre de pivotement.

La masse de la contre-masse 5 doit ainsi être inférieure à une limite définie par le bilan thermique de la micro-scie, sans nécessiter de configurer un système auto-équilibré où l'égalité (2)

$$M_c = M_s + M_p \qquad\qquad (2)$$

est strictement respectée.

La puissance thermique générée par le mouvement de va-et-vient de la contre-masse 5 sur l'élément de guidage 26 (tel que le premier rail de guidage 261 et le deuxième rail de guidage 262 parallèles à l'axe de transmission, c'est-à-dire le deuxième axe longitudinal B-B) est lié notamment aux facteurs suivants :

(i) la surface externe et la matière de la calotte de la micro-scie (le refroidissement dépendant essentiellement de la convection autour de la pièce-à-main 4);
(ii) le diamètre du manche de la micro-scie, qui impose le diamètre maximum de la contre-masse 5 et donc le bras de levier entre le centre de gravité de la contre-masse 5 et le premier doigt 241 de transmission du mouvement de va-et-vient ;
(iii) la fréquence f et la course D du mouvement de va-et-vient;
(iv) la distance de l'axe des rails de guidage 261,262 de la contre-masse 5 et l'axe de transmission du mouvement de va-et-vient, tel que le deuxième axe longitudinal B-B de l'arbre de transmission 22.

Une contre-masse plus lourde, telle que par exemple la contre-masse longue 5A, permet donc de mieux assurer la tenue du module micromoteur 10 avec la pièce-à-main 4, comprenant le module de transmission 2. Ceci provoque toutefois un bras de levier plus important entre son centre de gravité et le deuxième doigt 242 de transmission; par conséquent, pour minimiser la longueur de la pièce-à-main 4, il est préférable que la contre-masse 5 occupe tout l'espace libre à l'intérieur du manche, c'est-à-dire l'enveloppe extérieure de celle-ci qui est saisie par le chirurgien-dentiste. C'est ce qui explique la forme particulière de la contre-masse courte 5B de la figure 3 et de celle de la contre-masse longue 5A de la figure 4, dont la section correspondant substantiellement à un anneau cylindrique tronqué si l'on fait abstraction des orifices de guidage. Ainsi plus la contre-masse 5 est longue plus son centre de gravité se rapproche de l'axe central du manche; au contraire, l'extrémité du deuxième doigt 242 de transmission entraînant la contre-masse 5 est de préférence écartée au maximum du centre de la pièce à main 4 pour maximiser la course du mouvement de va-et-vient imposé à la contre-masse 5.

Les normes internationales fixent la température maximale qui peut être atteinte par la micro-scie (actuellement 55°C lors de l'utilisation normale), ce qui permet donc de déduire la puissance thermique maximale qui peut être générée par la présence de la contre-masse 5, laquelle sera réalisée de préférence en un matériau métallique tel que du bronze, du cuivre, de l'acier ou encore un métal noble et lourd de manière à présenter une densité supérieure à 7000 kg par mètre cube, et ainsi permettre d'obtenir une masse suffisante sans devoir requérir un volume trop important et encombrant vis-à-vis du reste du module de transmission, et en particulier la chaîne cinématique d'entraînement de l'outil de coupe 3 elle-même.

Le fait d'avoir, selon les modes de réalisation préférentiels décrits à l'aide de figures qui précèdent, des contre-masses guidées par au moins 2 rails de guidage permettent de réduire les frottements et le réchauffement.

Selon un mode de réalisation particulièrement avantageux, la masse de la contre-masse 5 selon l'invention est comprise entre le 25% et le 50% de la masse totale de l'ensemble outil de coupe 3 / porte-lame 27, ce qui permet de minimiser encore davantage tout réchauffement additionnel dû à l'auto-équilibrage. De manière encore plus préférée, la masse de la contre-masse 5 selon l'invention est comprise entre 2.5 g et 6 g, alors que la masse de l'outil de coupe est comprise entre 1 et 3 grammes.

Ainsi selon l'invention, il est proposé un moyen efficace de contrebalancer le déplacement du centre de masse d'un outil de coupe à l'aide d'une contre-masse qui:

- est indépendant de la vitesse de travail

- minimise la dépendance de la masse et de la forme des éléments constitutifs de la micro-scie à équilibrer

- minimise l'impact sur le rendement moyen (conversion mouvement rotatif-mouvement de va-et-vient) de la pièce-à-main

- minimise l'impact sur la vitesse d'usure des roulements

- ne génère pas de sur-réchauffement de la pièce à main

- permet une séparation physique de type modulaire entre la pièce-à-main 4 mécanique (le module formant la micro-scie, qui n'a pas besoin d'alimentation électrique), telle qu'illustrée sur la figure 1, et le moteur d'alimentation, comme le micro-moteur 10 illustré également sur la figure 1, en assurant que la connexion-déconnexion de l'outil de coupe chirurgicale au moteur soit simple et rapide, grâce à une force de déconnexion de l'ordre de 30 N (3 kg).

Selon les modes de réalisation préférentiels décrits, la présence de la contre-masse et d'un système de transmission 'symétrique' du mouvement rotatif à la contre-masse et à la lame permet de réduire les vibrations. Puisque la contre-masse bouge en phase parfaite avec la lame, la diminution des vibrations est assurée indépendamment de la vitesse de rotation du moteur. Par ailleurs, le guidage de la contre-masse permet d'éviter le réchauffement et l'usure potentiellement provoqués par l'ajout d'un composant lourd additionnel à la chaine de transmission. Le dédoublement des roulements à billes pour chaque doigt de transmission permet de réduire leur usure. Il est du reste possible de réduire la dépendance inverse du rendement de la puissance appliquée au moteur (le rendement diminue avec l'augmentation de puissance pour un système déséquilibré) en permettant d'augmenter l'efficacité de découpe.

Bien que seuls quelques modes de réalisation aient été décrits à titre d'exemple dans ce qui précède, on comprendra toutefois que ces derniers n'ont pas pour vocation d'exposer de manière exhaustive tous les modes de réalisation possibles. L'homme du métier comprendra qu'il est envisageable de remplacer un moyen décrit par un moyen équivalent sans sortir du cadre de la présente invention.

Par ailleurs, il serait également envisageable, sans sortir du cadre de la présente invention, de modifier la forme de la contre-masse en fonction de la géométrie de l'outil de coupe et du porte-lame utilisé, via un dispositif électronique ou électro-magnétique. Un mode de réalisation préférentiel pour implémenter une telle solution serait par exemple de solidariser deux parties de la contre-masse via un dispositif électromagnétique (du type électro-aimant) positionné sur une des deux parties de la contre-masse et un aimant positionné sur la deuxième partie de la contre-masse. Le changement de la polarité de l'électro-aimant permet donc soit de solidariser (attraction magnétique) soit de séparer (répulsion magnétique) les deux parties de la contre-masse. Si un outil de coupe plus lourd est utilisé, l'utilisateur peut imposer la solidarisation des deux parties (contre-masse 'lourde'), alors que si un outil de coupe plus léger est utilisé, l'utilisateur peut imposer la répulsion entre les deux parties de la contre-masse, et donc utiliser une contre-masse plus légère.

**Revendications**

1. Dispositif de découpage à usage chirurgical ou dentaire (1) comprenant un moteur (10) et une pièce à main (4) équipée d'un module de transmission (2) pour un outil de coupe (3), ledit moteur (10) étant relié de façon amovible à ladite pièce à main (4), ledit module de transmission (2) comprenant:

   - Une partie d'accouplement moteur agencée pour être entraînée en rotation par ledit moteur (10);
   - Une chaîne cinématique transformant ledit mouvement rotatif dudit moteur (10) en un premier mouvement de va-et-vient linéaire (M1) d'un porte-lame (27) via un arbre de transmission (22) rotatif pourvu d'un excentrique (23),

   ledit module de transmission (2) étant **caractérisé en ce qu'**il comprend par ailleurs une contre-masse (5) agencée de manière à se déplacer selon un deuxième mouvement de va-et-vient linéaire (M2) synchronisé sur ledit premier mouvement de va-et-vient linéaire (M1) dudit porte-lame (27), et dans le sens opposé à ce dernier.

2. Dispositif de découpage à usage chirurgical ou dentaire (1) selon la revendication 1, **caractérisé en ce que** la première chaîne cinématique comprend un premier doigt d'entraînement (241) dudit porte-lame (27) monté sur une première partie (231) dudit excentrique (23), ainsi qu'un deuxième doigt d'entraînement (242) de ladite contre-masse (5) monté sur une deuxième partie (232) dudit excentrique (23).

3. Dispositif de découpage à usage chirurgical ou dentaire (1) selon la revendication 2, ledit excentrique (23) étant agencé symétriquement en forme de « V », ledit premier doigt d'entraînement (241) dudit porte-lame (27) étant monté sur une première série de roulements (251) autour de ladite première partie (231) dudit excentrique (23) et ledit deuxième doigt d'entraînement (242) de ladite contre-masse (5) étant monté sur une deuxième série de roulements (252) autour de ladite deuxième partie (232) dudit excentrique (23).

4. Dispositif de découpage à usage chirurgical ou dentaire (1) selon l'une des revendications précédentes, dans lequel la forme géométrique de ladite contre-masse (5) est adaptée à celle dudit outil de coupe (3) et dudit porte-lame (27).

5. Dispositif de découpage à usage chirurgical ou dentaire (1) selon l'une des revendications précédentes, dans lequel ladite contre-masse (5) est guidée axialement selon un premier axe de transmission (A-A) correspondant vectoriellement au sens de déplacement longitudinal dudit porte-lame (27) et dudit outil de coupe (3) par au moins un élément de guidage axial (26) inséré dans au moins un orifice de guidage (52) pratiqué dans ladite contre-masse (5).

6. Dispositif de découpage à usage chirurgical ou dentaire (1) selon la revendication 5, dans lequel ledit élément de guidage axial (26) est formé par un premier rail de guidage axial (261) et un deuxième rail de guidage axial (262) reliant parallèlement une première pièce (201) du châssis (20) connectée audit moteur (10) à une deuxième pièce (202) du châssis (20) supportant et guidant axialement ledit porte-lame (27) dudit module de transmission (2), ledit premier rail de guidage axial (261) et ledit deuxième rail de guidage axial (262) étant insérés dans des orifices de guidage (52) respectifs de ladite contre-masse (5).

7. Dispositif de découpage à usage chirurgical ou dentaire (1) selon l'une des revendications précédentes, **caractérisé en ce que** ledit module de transmission (2) comprend par ailleurs un dispositif de refroidissement (7) pour la contre-masse (5).

8. Dispositif de découpage à usage chirurgical ou dentaire (1) selon la revendication 7, lorsqu'elle dépend de la revendication 6, **caractérisé en ce que** le premier rail de guidage axial (261) comprend un premier conduit de refroidissement intégré (2610) et/ou que ledit deuxième rail de guidage axial (262) comprend un deuxième conduit de refroidissement intégré (2620).

9. Dispositif de découpage à usage chirurgical ou dentaire (1) selon l'une des revendications précédentes, **caractérisé en ce que** ledit module de transmission (2) est connecté de façon amovible audit moteur (10), et que le poids de ladite contre-masse (5) est déterminé de manière à être supérieur à une valeur minimale définie par rapport au poids combiné de l'ensemble formé par le porte-lame (27) et dudit outil de coupe (3) en fonction de la force de déconnexion (Fd) dudit module de transmission (2) audit moteur (10), de la course (D) dudit outil de coupe (3), et de la fréquence (f) d'oscillation du mouvement de va-et-vient dudit outil de coupe (3).

10. Dispositif de découpage à usage chirurgical ou dentaire (1) selon l'une des revendications précédentes, **caractérisé**

**en ce que** le poids de ladite contre-masse (5) est déterminé de manière à être inférieur à une valeur prédéfinie déterminée par un bilan thermique.

11. Dispositif de découpage à usage chirurgical ou dentaire (1) selon l'une des revendications 8 à 10, lorsqu'elles dépendent de la 7, **caractérisé en ce que** le poids de ladite contre-masse (5) est compris entre 25% et 50% du poids combiné de l'ensemble formé par ledit porte-lame (27) et ledit outil de coupe (3).

12. Dispositif de découpage à usage chirurgical ou dentaire (1) selon l'une des revendications 7 à 11 précédentes, **caractérisée en ce que** la densité de ladite contre-masse (5) est supérieure à 7000 kg par mètre cube.

13. Dispositif de découpage à usage chirurgical ou dentaire (1) selon l'une des revendications précédentes, **caractérisé en ce que** la partie d'accouplement moteur dudit module de transmission (2) consiste en un orifice de couplage (21) cannelé, lequel coopère avec un nez d'accouplement (11) standard dudit moteur (10) comportant une série de rainures (110) dans lesquelles sont insérées des O-rings (111).

14. Pièce à main (4) comprenant un module de transmission (2) pour dispositif de découpage à usage chirurgical ou dentaire (1) selon l'une des revendications 1 à 13 précédentes.

**Patentansprüche**

1. Schneidvorrichtung für chirurgische oder zahnärztliche Zwecke (1), umfassend einen Motor (10) und ein Handstück (4), welches Handstück (4) mit einem Übertragungsmodul (2) für ein Schneidwerkzeug (3) ausgestattet ist, und wobei der Motor (10) abnehmbar mit dem Handstück (4) verbunden ist, und wobei das Übertragungsmodul (2) umfasst:

   - ein Kupplungselement, das derart angeordnet ist, dass es durch den Motor (10) gedreht wird;
   - eine kinematische Kette, die die Drehbewegung des Motors (10) über eine mit einem Exzenter (23) versehene Welle (22) in eine erste lineare Hubbewegung (M1) eines Klingenhalters (27) umwandelt,
   wobei das Übertragungsmodul (2) **dadurch gekennzeichnet ist, dass** es außerdem eine Gegenmasse (5) umfasst, die derart angeordnet ist, dass sie entlang einer zweiten linearen Hubbewegung (M2) verschoben wird, welche zweite lineare Hubbewegung (M2) mit der ersten linearen Hubbewegung (M1) des Klingenhalters (27) synchronisiert ist und in entgegengesetzter Richtung zu dieser verläuft.

2. Schneidvorrichtung für chirurgische oder zahnärztliche Zwecke (1) gemäß Anspruch 1, **dadurch gekennzeichnet, dass** die erste kinematische Kette einen ersten Antriebsfinger (241) des Klingenhalters (27), der an einem ersten Teil (231) des Exzenters (23) angebracht ist, sowie einen zweiten Antriebsfinger (242) der Gegenmasse (5), der an einem zweiten Teil (232) des Exzenters (23) angebracht ist, umfasst.

3. Schneidvorrichtung für chirurgische oder zahnärztliche Zwecke (1) gemäß Anspruch 2, wobei der Exzenter (23) symmetrisch in einer "V"-Form angeordnet ist, wobei der erste Antriebsfinger (241) des Klingenhalters (27) auf einer ersten Reihe von Lagern (251) um den ersten Teil (231) des Exzenters (23) montiert ist und der zweite Antriebsfinger (242) des Gegenmasse (5) auf einer zweiten Reihe von Lagern (252) um den zweiten Teil (232) des Exzenters (23) montiert ist.

4. Schneidvorrichtung für chirurgische oder zahnärztliche Zwecke (1) gemäß einem der vorhergehenden Ansprüche, wobei die geometrische Form der Gegenmasse (5) an diejenige des Schneidwerkzeugs (3) und des Klingenhalters (27) angepasst ist.

5. Schneidvorrichtung für chirurgische oder zahnärztliche Zwecke (1) gemäß einem der vorhergehenden Ansprüche, wobei die Gegenmasse (5) durch mindestens ein axiales Führungselement (26), welches in mindestens ein in der Gegenmasse (5) ausgebildetes Führungsloch (52) eingesetzt ist, axial entlang einer ersten Übertragungsachse (A-A) geführt wird, welche erste Übertragungsachse (A-A) vektoriell der Richtung der Längsverschiebung des Klingenhalters (27) und des Schneidwerkzeugs (3) entspricht.

6. Schneidvorrichtung für chirurgische oder zahnärztliche Zwecke (1) gemäß Anspruch 5, wobei das axiale Führungselement (26) durch eine erste axiale Führungsschiene (261) und eine zweite axiale Führungsschiene (262) gebildet wird, die parallel ein erstes Teil (201) des Rahmens (20), das mit dem Motor (10) verbunden ist, mit einem zweiten Teil (202) des Rahmens (20) verbindet, das den Klingenhalter (27) des Übertragungsmoduls (2) trägt und axial führt, wobei die erste axiale Führungsschiene (261) und die zweite axiale Führungsschiene (262) in jeweilige Führungslöcher (52) der Gegenmasse (5) eingesetzt sind.

7.  Schneidvorrichtung für chirurgische oder zahnärztliche Zwecke (1) gemäß einem der vorhergehenden Ansprüche, **dadurch gekennzeichnet, dass** das Übertragungsmodul (2) ferner eine Kühlvorrichtung (7) für die Gegenmasse (5) umfasst.

8.  Schneidvorrichtung für chirurgische oder zahnärztliche Zwecke (1) gemäß Anspruch 7, insofern sie von Anspruch 6 abhängig ist, **dadurch gekennzeichnet, dass** die erste axiale Führungsschiene (261) einen ersten integrierten Kühlkanal (2610) umfasst und/oder dass die zweite axiale Führungsschiene (262) einen zweiten integrierten Kühlkanal (2620) umfasst.

9.  Schneidvorrichtung für chirurgische oder zahnärztliche Zwecke (1) nach einem der vorhergehenden Ansprüche, **dadurch gekennzeichnet, dass** das Übertragungsmodul (2) abnehmbar mit dem Motor (10) verbunden ist, und dass das Gewicht der Gegenmasse (5) so bestimmt ist, dass es größer ist als ein Minimalwert, der in Bezug auf das kombinierte Gewicht der durch den Klingenhalter (27) und das Schneidwerkzeug (3) gebildeten Baugruppe, der Funktion der Trennkraft (Fd) des Übertragungsmoduls (2) zum Motor (10), des Verlaufs (D) des Schneidwerkzeugs (3) und der Frequenz (f) der Schwingung der Hubbewegung des Schneidwerkzeugs (3) definiert ist.

10. Schneidvorrichtung für chirurgische oder zahnärztliche Zwecke (1) gemäß einem der vorhergehenden Ansprüche, **dadurch gekennzeichnet, dass** das Gewicht der Gegenmasse (5) so bestimmt ist, dass es kleiner als ein durch eine Wärmebilanz bestimmter Wert ist.

11. Schneidvorrichtung für chirurgische oder zahnärztliche Zwecke (1) gemäß einem der Ansprüche 8 bis 10, insofern sie von Anspruch 7 abhängig sind, **dadurch gekennzeichnet, dass** das Gewicht der Gegenmasse (5) zwischen 25 % und 50 % des kombinierten Gewichts der durch den Klingenhalter (27) und das Schneidwerkzeug (3) gebildeten Baugruppe beträgt.

12. Schneidvorrichtung für chirurgische oder zahnärztliche Zwecke (1) gemäß einem der vorhergehenden Ansprüche 7 bis 11, **dadurch gekennzeichnet, dass** die Dichte der Gegenmasse (5) größer als 7000 kg pro Kubikmeter ist.

13. Schneidvorrichtung für chirurgische oder zahnärztliche Zwecke (1) gemäß einem der vorhergehenden Ansprüche, **dadurch gekennzeichnet, dass** das Kupplungselement des Übertragungsmoduls (2) aus einer geriffelten Kupplungsöffnung (21) besteht, die mit einer Standardkupplungsnase (11) des Motors (10) zusammenwirkt, die eine Reihe von Nuten (110) aufweist, in welche Nuten (110) O-Ringe (111) eingesetzt sind.

14. Handstück (4) umfassend ein Übertragungsmodul (2) für die Schneidvorrichtung für chirurgische oder zahnärztliche Zwecke (1) gemäß einem der vorhergehenden Ansprüche 1 bis 13.

**Claims**

1.  A cutting device for surgical or dental use (1) comprising a motor (10) and a handpiece (4) equipped with a transmission module (2) for a cutting tool (3), said motor (10) being detachably connected to said handpiece (4), said transmission module (2) comprising:

    - a motor coupling part arranged to be rotated by said motor (10);
    - a kinematic chain transforming said rotary movement of said motor (10) into a first linear reciprocating movement (M1) of a blade holder (27) via a rotary transmission shaft (22) provided with an eccentric (23),

    said transmission module (2) being **characterized in that** it further comprises a counter-mass (5) arranged so as to be displaced according to a second linear reciprocating movement (M2) synchronized with said first linear reciprocating movement (M1) of said blade holder (27), and in the opposite direction to the latter.

2.  The cutting device for surgical or dental use (1) according to Claim 1, **characterized in that** the first kinematic chain comprises a first drive finger (241) of said blade holder (27) mounted on a first part (231) of said eccentric (23), as well as a second drive finger (242) of said counter-mass (5) mounted on a second part (232) of said eccentric (23).

3. The cutting device for surgical or dental use (1) according to Claim 2, said eccentric (23) being symmetrically arranged in a "V" shape, said first drive finger (241) of said blade holder (27) being mounted on a first series of bearings (251) around said first part (231) of said eccentric (23) and said second drive finger (242) of said counter-mass (5) being mounted on a second series of bearings (252) around said second part (232) of said eccentric (23).

4. The cutting device for surgical or dental use (1) according to any one of the preceding claims, wherein the geometric shape of said counter-mass (5) is adapted to that of said cutting tool (3) and of said blade holder (27).

5. The cutting device for surgical or dental use (1) according to any one of the preceding claims, wherein said counter-mass (5) is guided axially along a first transmission axis (A-A) corresponding vectorially to the direction of longitudinal displacement of said blade holder (27) and of said cutting tool (3) by at least one axial guide element (26) inserted into at least one guide hole (52) made in said counter-mass (5).

6. The cutting device for surgical or dental use (1) according to Claim 5, wherein said axial guide element (26) is formed by a first axial guide rail (261) and a second axial guide rail (262) connecting, in parallel, a first piece (201) of the frame (20) connected to said motor (10) to a second piece (202) of the frame (20) supporting and axially guiding said blade holder (27) of said transmission module (2), said first axial guide rail (261) and said second axial guide rail (262) being inserted into respective guide holes (52) of said counter-mass (5).

7. The cutting device for surgical or dental use (1) according to any one of the preceding claims, **characterized in that** said transmission module (2) further comprises a cooling device (7) for the counter-mass (5).

8. The cutting device for surgical or dental use (1) according to Claim 7, when it is dependent on Claim 6, **characterized in that** the first axial guide rail (261) comprises a first integrated cooling duct (2610) and/or **in that** said second axial guide rail (262) comprises a second integrated cooling duct (2620).

9. The cutting device for surgical or dental use (1) according to any one of the preceding claims, **characterized in that** said transmission module (2) is detachably connected to said motor (10), and **in that** the weight of said counter-mass (5) is determined so as to be greater than a minimum value defined with respect to the combined weight of the assembly formed by the blade holder (27) and of said cutting tool (3) as a function of the disconnection force (Fd) of said transmission module (2) to said motor (10), of the course (D) of said cutting tool (3), and of the frequency (f) of oscillation of the reciprocating movement of said cutting tool (3).

10. The cutting device for surgical or dental use (1) according to any one of the preceding claims, **characterized in that** the weight of said counter-mass (5) is determined so as to be less than a predefined value determined by a heat balance.

11. The cutting device for surgical or dental use (1) according to any one of Claims 8 to 10, when they are dependent on Claim 7, **characterized in that** the weight of said counter-mass (5) is between 25% and 50% of the combined weight of the assembly formed by said blade holder (27) and said cutting tool (3).

12. The cutting device for surgical or dental use (1) according to any one of the preceding Claims 7 to 11, **characterized in that** the density of said counter-mass (5) is greater than 7000 kg per cubic meter.

13. The cutting device for surgical or dental use (1) according to any one of the preceding claims, **characterized in that** the motor coupling part of said transmission module (2) consists of a fluted coupling hole (21) which cooperates with a standard coupling nose (11) of said motor (10) having a series of grooves (110) into which O-rings (111) are inserted.

14. A handpiece (4) comprising a transmission module (2) for the cutting device for surgical or dental use (1) according to any one of the preceding Claims 1 to 13.

FIG. 1

FIG. 2B

FIG. 2A

**FIG. 3**

EP 4 208 121 B1

FIG. 4

**FIG. 5**

FIG. 6

FIG. 7a

FIG. 7b

FIG. 8

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4036236 A
- US 5725530 A
- WO 9710765 A
- US 2015066032 A
- US 3642002 A
- EP 2316356 A